# EUROPÄISCHE PATENTSCHRIFT

(11) **EP 0 410 169 B1**
(45) Veröffentlichungstag und Bekanntmachung des Hinweises auf die Patenterteilung: **14.12.1994**
(21) Anmeldenummer: 90112743.1
(22) Anmeldetag: 04.07.1990
(51) Int. Cl.: C07C 273/18, C07C 275/06, C07C 269/00, C07C 263/00, C07C 333/04, C07C 265/04

(54) **Verfahren zur Herstellung unsymmetrisch substituierter Harnstoffe, Carbamate, Thiocarbamate und substituierter Isocyanate**
Process for the preparation of asymmetrically substituted ureas, carbamates, thiocarbamates and substituted isocyanates
Procédé de préparation d'urées substituées asymétriquement, carbamates, thiocarbamates et isocyanates substitués

(30) Priorität: 28.07.1989 AT 1831/89; 28.07.1989 AT 1832/89; 28.07.1989 AT 1833/89
(43) Veröffentlichungstag der Anmeldung: 30.01.1991
(73) Patentinhaber: Chemie Linz GmbH, A-4021 Linz (AT)
(72) Erfinder: Müllner, Martin, Dipl.-Ing. Dr., A-4050 Traun (AT); Stern, Gerhard, Dipl.-Ing. Dr., A-4180 Sonnberg (AT); Erich, Schulz, A-4052 Ansfelden (AT); Rössler, Markus, A-4020 Linz (AT)
(74) Vertreter: Kunz, Ekkehard, Dr.

(56) Entgegenhaltungen:
- EP-A- 0 124 704
- JUSTUS LIEBIGS ANNALEN DER CHEMIE, Bd. 364, 1909, WEINHEIM, DE, Seiten 129 - 146; A. MICHAEL et al: "Zur Constitution der Cyansäure"

## Beschreibung

Die vorliegende Erfindung betrifft die Herstellung unsymmetrisch substituierter Harnstoffe, Carbamate, Thiocarbamate und substituierter Isocyanate durch Umsetzung eines Adduktes aus Isocyansäure und einem tertiären Amin in einem Verdünnungsmittel mit primären oder sekundären Aminen, Alkoholen, Thiolen oder einer Verbindung, die eine oder zwei nicht kumulierte, olefinische Doppelbindungen aufweist.

Die Herstellung unsymmetrisch substituierter Harnstoffe kann gemäß Liebig's Annalen der Chemie, 364. Band, Seiten 129 bis 146 durch Umsetzung von reiner Isocyansäure mit einem primären oder sekundären Amin in einem Lösungsmittel erfolgen. Carbamate können nach Houben-Weyl, Methoden der organischen Chemie, Erweiterungs- und Folgebände, Band E4, Seiten 181 bis 189 durch Umsetzung von Isocyanaten mit Alkoholen hergestellt werden. Aus DD 116551 ist bekannt, daß Isocyansäure mit Isopropenylbenzol in einem organischen Lösungsmittel zum entsprechenden Isocyanat umgesetzt werden kann. Lösungen freier Isocyansäure sind aber technisch nur aufwendig herstellbar und schwierig zu handhaben, sodaß sie großtechnisch nur beschränkt eingesetzt werden können und aus Isocyanaten muß die Isocyansäure für die Umsetzung freigesetzt werden, wobei es leicht zu Polymerisierungsreaktionen kommt.

Es wurde nun gefunden, daß bei Zugabe von primären oder sekundären Aminen, von Alkoholen, von Thiolen oder von Verbindungen, die eine oder zwei nicht kumulierte olefinische Doppelbindungen aufweisen, zu einer Lösung oder Suspension eines Adduktes von Isocyansäure mit einem tertiären Amin, die technisch relativ einfach erhalten werden kann, unsymmetrisch substituierte Harnstoffe, Carbamate, Thiocarbamate oder substituierte Isocyanate erhalten werden. Unerwarteterweise muß die Isocyansäure aus dem Addukt nicht durch Zugabe einer Säure in Freiheit gesetzt werden. Das Addukt verhält sich vielmehr wie die freie Isocyansäure selbst.

Gegenstand der Erfindung ist daher ein Verfahren zur Herstellung unsymmetrisch substituierter Harnstoffe, Carbamate, Thiocarbamate oder substituierter Isocyanate, das dadurch gekennzeichnet ist, daß ein Addukt aus Isocyansäure und einem tertiären Amin in einem unter den Reaktionsbedingungen inerten Verdünnungsmittel mit einem primären oder sekundären Amin, einem Alkohol, einem Thiol oder einer Verbindung, mit einer oder zwei nicht kumulierten olefinischen Doppelbindungen, umgesetzt wird.

Geeignete Addukte aus Isocyansäure und einem tertiären Amin sind substituierte Ammoniumisocyanate der Formel R₁R₂R₃N.HNCO, in der die Reste R₁, R₂ und R₃ einen cyclischen Aminteil bedeuten, wie z.B. N-Alkylpyrrolidin, N-Alkylpyrrol, N-Alkylpiperidin, Pyridin, N-Alkyl-Morpholin oder R₁, R₂ und R₃ bedeuten unabhängig voneinander geradkettige oder verzweigte Alkyl-, Aryl-, Alkylaryl- oder Arylalkylgruppen. Geradkettige oder verzweigte Alkylgruppen sind beispielsweise Alkylgruppen mit 1 bis 10 C-Atomen, wie Methyl-, Ethyl-, Propyl-, Butylgruppen und ihre Isomeren, wie iso-Propyl-, iso-Butyl-, tert. Butylgruppen. Aryl-, Alkylaryl- oder Arylalkylgruppen sind gegebenenfalls ein oder mehrfach durch geradkettige oder verzweigte Alkylgruppen mit 1 bis 5 C-Atomen substituierte Phenylgruppen, die mit dem Stickstoffatom entweder über ein aromatisches oder über ein aliphatisches Kohlenstoffatom verbunden sein können. Beispiele solcher Gruppen sind etwa Phenyl-, Tolyl-, Dimethylphenyl-, Trimethylphenyl-, Ethylphenyl-, Isopropylphenyl-, Benzyl-, Methylbenzyl-, Ethylenphenylgruppen. Bevorzugt sind Addukte mit tertiären Aminen der allgemeinen Formel NR₁R₂R₃, in denen R₁, R₂ und R₃ gleich sind und eine Alkylgruppe bedeuten. Besonders bevorzugt sind dabei Alkylgruppen mit 1 bis 5 C-Atomen, beispielsweise Trimethylamin, Triethylamin, Tripropylamin, Tributylamin und Triisopentylamin. Ganz besonders bevorzugt sind Trimethylamin, Triethylamin und Triisopentylamin.

Das Addukt aus Isocyansäure und tertiärem Amin kann beispielsweise aus einem gasförmigen Gemisch aus Isocyansäure und Ammoniak hergestellt werden, indem man diesem Gemisch, das eine Temperatur von 250 bis 600 °C aufweist, ein tertiäres Amin zusetzt, das entstandene gasförmige Reaktionsgemisch mit einem inerten Verdünnungsmittel in Kontakt bringt und abkühlt. Das nötige Ausgangsprodukt, das gasförmige Gemisch aus Isocyansäure und Ammoniak entsteht bei der thermischen Zersetzung von Harnstoff, beispielsweise nach EP-A-0 124 704.

Zur Herstellung der Verbindungen gemäß er Erfindung wird zuerst das Addukt aus Isocyansäure und tertiärem Amin in einem unter den Reaktionsbedingungen inerten Verdünnungsmittel bei Temperaturen von etwa -20°C bis Raumtemperatur vorgelegt. Anschließend wird unter Rühren ein primäres oder sekundäres Amin, ein Alkohol, ein Thiol oder eine Verbindung, die eine oder zwei nicht kumulierte olefinische Doppelbindungen aufweist, zugegeben.

Als inerte Verdünnungsmittel kommen beispielsweise aliphatische Kohlenwasserstoffe, wie Pentan, Hexan, Heptan, aromatische Kohlenwasserstoffe, wie Benzol, Toluol, Xylol, halogenierte aliphatische Kohlenwasserstoffe, wie Methylenchlorid, Chloroform, Tetrachlorkohlenstoff, Ethylchlorid, Ethylenchlorid, halogenierte aromatische Kohlenwasserstoffe, wie Chlorbenzol, Trichlorbenzol, Ether, wie Diethylether, Diisopropylether, Dibutylether, Ethylmethylether, Dioxan, Carbonsäureamide, wie Dimethylformamid, N-Methylpyrrolidon oder Mischungen von oben angeführten Verdünnungsmitteln in Frage. Bevorzugt sind aromatische Kohlenwasserstoffe, halogenierte aliphatische Kohlenwasserstoffe und Carbonsäureamide, besonders bevorzugt sind Toluol, Chloroform, oder N-Methylpyrrolidon.

Unter primären und sekundären Aminen sind solche Verbindungen zu verstehen, die eine oder mehrere Aminogruppen aufweisen. Sie können gegebenenfalls durch weitere, unter den Reaktionsbedingungen inerte Gruppen substituiert sein. Beispiele hiefür sind aliphatische, cycloaliphatische oder cyclische Amine , wie Methylamin, Ethylamin, Hexylamin, Hexadecylamin, Isopropylamin, Isobutylamin, Isooctylamin, Methylethylamin, Cyclohexylamin, Pyrrolidin, Pyrrol, Piperidin, Morpholin oder Dimethylamin, Diethylamin, Diisopropylamin, Ethylendiamin, Hexamethylendiamin, 4,4′-Diaminodicyclohexylmethan oder aromatische Amine, wie Anilin, Nitroaniline, Chloraniline, Tolylamine, Benzylamin, Naphthylamine, Phenylendiamine, Toluylendiamine, 4,4′-Diaminodiphenylmethan.

Unter Alkoholen oder Thiolen sind Verbindungen zu verstehen, die eine oder mehrere Hydroxyl- oder Mercaptogruppen aufweisen. Sie können gegebenenfalls durch weitere, unter Reaktionsbedingungen inerte Gruppen substituiert sein. Beispiele für solche Verbindungen sind aliphatische oder cycloaliphatische Alkohole oder Thiole, wie Methanol, Ethanol, Propanol, Octadecylalkohol, Isopropanol, Isooctanol, Cyclohexanol, Cyclooctanol, Ethylenglykol, Glycerin, Methylmercaptan, Ethylmercaptan, Isooctylmercaptan, Ethandithiol, Thioglykol, oder aromatische Alkohole oder Thiole, wie Phenol, Nitrophenole, Chlorphenole, Naphthole, Benzylalkohole, Resorcin, Thiophenol, Bisphenol A, Polyesterole, Polyetherole.

Verbindungen, die eine oder mehrere olefinische Doppelbindungen aufweisen, die gegebenenfalls durch weitere unter Reaktionsbedingungen inerte Gruppen substituiert sein können, sind beispielsweise aliphatische oder cycloaliphatische Verbindungen, wie Ethen, Propen, Buten, Penten, Hexen, Hexadecen, Isopropen, Isobuten, Isoocten, Cyclohexen, Butadien, Octadien, Cyclooctadien, Isopren, Terpene, oder aromatische Verbindungen mit olefinischer Doppelbindung, wie beispielsweise Styrole, Divinylbenzole, Diisopropenylbenzol, Naphthylstyrole, Diphenylethylene.

Das Amin, der Alkohol, das Thiol oder das Olefin kann als solches, gasförmig oder flüssig, oder zusammen mit einem unter den Reaktionsbedingungen inerten wie oben beschriebenen gasförmigen oder flüssigen Verdünnungsmittel zugegeben werden.
Das Amin, der Alkohol, das Thiol oder das Olefin kann in äquivalenter Menge oder in einem Überschuß zum Addukt aus Isocyansäure und tertiärem Amin zugegeben werden. Es kann aber auch zweckmäßig sein, die Isocyansäure im Überschuß zuzusetzen, um den Ablauf der Reaktion zu verbessern.

Bevorzugt werden pro Mol des Adduktes aus Isocyansäure und tertiärem Amin 1 bis 7, besonders bevorzugt 1 bis 3 Mol Äquivalente Amin, Alkohol, Thiol oder Olefin zugegeben.

Nach beendeter Zugabe des Amins, des Alkohols, des Thiols oder des Olefins wird zur Vervollständigung der Reaktion bei Raumtemperatur nachgerührt und/oder gegebenenfalls bis auf Rückflußtemperatur des verwendeten Verdünnungsmittels erhitzt. Gegebenenfalls erfolgt die Reaktion auch unter Druck, wobei Drücke bis zu 20 bar angewendet werden können. Nach dem Abkühlen kristallisiert die gebildete Verbindung aus dem Verdünnungsmittel aus und wird abfiltriert, oder das Verdünnungsmittel wird verdampft. Gegebenenfalls kann auf übliche Weise, wie z.B. durch Umkristallisation, Destillation, Chromatographie, eine weitere Reinigung erfolgen.

Das erfingungsgemäße Verfahren liefert unsymmetrisch substituierte Harnstoffe, Carbamate oder Thiocarbamate, substituierte Isocyanate in guter Reinheit und hohen Ausbeuten und stellt somit eine Bereicherung der Technik dar.

### Beispiel 1

### Herstellung von Triethylammoniumisocyanat

100 g Harnstoff wurden pro Stunde kontinuierlich in einen Zersetzer eingetragen. Die Pyrolysegase wurden in einem beheizbaren Rohr bei 320°C mit 255 g Triethylamin pro Stunde, das gasförmige eingebracht wurde, zur Reaktion gebracht. Die Reaktionsgase wurden in einem Wäscher, der mit Chloroform betrieben wurde, rasch auf Raumtemperatur abgekühlt.
Insgesamt wurde 213 g (3,5 Mol) Harnstoff und 544 g (5,4 Mol) Triethylamin eingetragen.
Dabei wurde **Triethylammoniumisocyanat** in einer Ausbeute von 66 % der Theorie, gelöst in Chloroform, erhalten.

IR: 2160 cm⁻¹ (scharfe Bande)

### Beispiel 2

In 100 ml einer Lösung von 10 g Triethylammoniumisocyanat (0,069 Mol) in Chloroform, hergestellt nach Beispiel 1, wurden bei Raumtemperatur 14,1 g Dodecylamin (0,076 Mol) gelöst in 20 ml Chloroform unter Rühren zugetropft. Nach beendeter Zugabe wurde 24 Stunden bei Raumtemperatur nachgerührt und 1 Stunde auf Rückfluß erhitzt. Das Lösungsmittel wurde abgedampft und der Rückstand aus Chloroform umkristallisiert. Dabei wurden 9,45 g, das sind 60 % der Theorie, **Dodecylharnstoff** erhalten.

| | | | | |
|---|---|---|---|---|
| C-H-N-Analyse: | theoret.: | C 68,4 %, | H 12,3 %, | N 12,3 % |
| | gef.: | C 68,2 %, | H 12,3 %, | N 12,3 % |

### Beispiel 3

Wie im Beispiel 2 beschrieben, aber unter Verwendung von 4,9 g iso-Propylamin (0,083 Mol) und 100 ml Chlorbenzol als Lösungsmittel wurde nach Umkristallisieren aus Wasser **iso-Propylharnstoff** in einer Ausbeute von 80 % der Theorie erhalten.

| | | | | |
|---|---|---|---|---|
| C-H-N-Analyse: | theoret.: | C 46,7 %, | H 9,8 %, | N 27,2 % |
| | gef.: | C 47,0 %, | H 9,6 %, | N 27,4 % |

### Beispiel 4

Wie im Beispiel 2 beschrieben, aber unter Verwendung von 7,0 g Cyclohexylamin (0,071 Mol) wurde nach dem Umkristallisieren aus Wasser **Cyclohexylharnstoff** in einer Ausbeute von 70 % der Theorie erhalten.

| | | | | |
|---|---|---|---|---|
| C-H-N-Analyse: | theoret.: | C 59,1 %, | H 9,9 %, | N 19,7 % |
| | gef.: | C 59,2 %, | H 9,9 %, | N 19,7 % |

### Beispiel 5

In 50 ml einer Lösung von 6,2 g (0,043 Mol) Triethylammoniumisocyanat in Chloroform, hergestellt nach Beispiel 1, wurden 6,3 g (0,086 Mol) Diethylamin eingetropft, sodaß die Temperatur nicht über Raumtemperatur anstieg. Nach beendeter Zugabe wurde 24 Stunden bei Raumtemperatur nachgerührt und anschließend 30 Minuten unter Rückfluß erhitzt. Das Reaktionsgemisch wurde abgedampft und der Rückstand aus Diisopropylether umkristallisiert, wobei 3,5 g, das sind 70 % der Theorie **N,N-Diethylharnstoff** mit einem Schmelzpunkt von 69 - 71°C erhalten wurde.

### Beispiel 6

In 100 ml einer Lösung von 10,6 g (0,0735 Mol) Triethylammoniumisocyanat in Chloroform, hergestellt nach Beispiel 1, wurden bei Raumtemperatur unter Rühren 13,7 g (0,147 Mol) Anilin zugetropft. Nach beendeter Zugabe wurde 24 Stunden bei Raumtemperatur nachgerührt, worauf 30 Minuten auf Rückfluß erhitzt wurde. Der beim Abkühlen der Reaktionsmischung ausgefallene Niederschlag wurde abgesaugt und mit wenig Diethylether nachgewaschen. Durch Einengen der Mutterlauge wurde eine zweite Kristallfraktion erhalten.
Insgesamt wurden 6,0 g, das sind 60 % der Theorie **Phenylharnstoff** mit einem Schmelzpunkt von 143 bis 145°C erhalten.
Nach Umkristallisieren einer kleinen Menge aus Wasser betrug der Schmelzpunkt 146 - 148°C.

### Beispiel 7

In 100 ml einer Lösung von 10,1 g (0,0542 Mol) Tri-n-propylammoniumisocyanat, hergestellt nach der im Beispiel 1 beschriebenen Vorgangsweise, wurden bei -10°C 10,1 g (0,1084 Mol) Anilin, gelöst in 10 ml Chloroform, zugesetzt. Nach beendeter Zugabe wurde 24 Stunden bei Raumtemperatur nachgerührt und 30 Minuten auf Rückfluß erhitzt. Nach dem Abkühlen der Reaktionsmischung kristallisierte Phenylharnstoff aus, der abgesaugt, mit wenig Diethylether nachgewaschen und getrocknet wurde. Dabei wurden 4,8 g **Phenylharnstoff**, das enspricht 65 % der Theorie, mit einem Schmelzpunkt von 144 bis 146°C erhalten.

### Beispiel 8

In 160 ml einer Lösung von 16,5 g (0,0722 Mol) Tri-n-butylammoniumisocyanat in Chloroform, hergestellt nach der in Beispiel 1 beschriebenen Vorgangsweise, wurden 13,5 g (0,1444 Mol) Anilin bei Raumtemperatur eingetropft. Nach beendeter Zugabe wurde 24 Stunden bei Raumtemperatur gerührt und anschließend 30 Minuten auf Rückfluß erhitzt. Nach dem Abkühlen fiel Phenylharnstoff aus, der abgesaugt, mit wenig Diethylether gewaschen und getrocknet wurde. Dabei wurden 5,9 g **Phenylharnstoff**, das entspricht 60 % der Theorie, mit einem Schmelzpunkt von 142 bis 145°C erhalten.

### Beispiel 9

In 130 ml einer Lösung von 17,2 g (0,0636 Mol) Triisopentylammoniumisocyanat in Chloroform, hergestellt nach der in Beispiel 1 beschriebenen Vorgangsweise, wurden 11,8 g (0,1267 Mol) Anilin eingetropft, sodaß die Temperatur nicht über Raumtemperatur anstieg. Nach beendeter Zugabe wurde 24 Stunden bei Raumtemperatur nachgerührt und anschließend 30 Minuten auf Rückfluß erhitzt. Nach dem Abkühlen fiel Phenylharnstoff aus, der abgesaugt, mit wenig Ether gewaschen und getrocknet wurde. Dabei wurden 5,1 g **Phenylharnstoff**, das entspricht 60 % der Theorie, mit einem Schmelzpunkt von 143 - 145°C erhalten.

### Beispiel 10

Wie im Beispiel 6 beschrieben, aber unter Verwendung von Diethylether als Lösungsmittel wurde nach dem Umkristallisieren aus Wasser **Phenylharnstoff** mit einem Schmelzpunkt von 146 bis 147 °C in einer Ausbeute von 50 % der Theorie erhalten.

### Beispiel 11

Wie im Beispiel 6 beschrieben, aber unter Verwendung von 9,4 g 4-Chloranilin (0,0735 Mol) und Dimethoxyethan als Lösungsmittel wurde **4-Chlor-phenylharnstoff** in einer Ausbeute von 50 % der Theorie erhalten.
¹H-NMR: 6,8-7,0 (s, breit, -NH₂); 7,36 (d, aromat. -CH-); 7,51 (d, aromat. -CH-); 9.0 (s, -NH-).

### Beispiel 12

In 100 ml einer Lösung von 10 g Triethylammoniumisocyanat (0,069 Mol) in 100 ml N-Methylpyrrolidon, hergestellt nach der im Beispiel 1 beschriebenen Vorgangsweise, wurde bei Raumtemperatur 1,88 g Ethylendiamin (0,031 Mol) unter Rühren getropft. Nach 24 Stunden Rühren bei Raumtemperatur wurde die Reaktionsmischung eine Stunde auf Rückfluß erhitzt, das Lösungsmittel abgedampft und der Rückstand aus Wasser umkristallisiert. Dabei wurden 3,4 g, das sind 75 % der Theorie **Ethylendiharnstoff** erhalten.

| | | | | |
|---|---|---|---|---|
| C-H-N-Analyse: | theoret.: | C 32,9 %, | H 6,9 %, | N 38,3 % |
| | gef.: | C 32,8 %, | H 7,0 %, | N 38,2 % |

### Beispiel 13

In 100 ml einer Lösung von 10,5 g (0,073 Mol) Triethylammomiumisocyanat in Chloroform, hergestellt nach Beispiel 1, wurden 6,7 g (0,146 Mol) Ethanol bei Raumtemperatur unter Rühren getropft, worauf 24 Stunden bei Raumtemperatur nachgerührt und anschließend 1 Stunde auf Rückfluß erhitzt wurde.
Das Lösungsmittel wurde abgedampft und der Rückstand aus Ethanol umkristallisiert, wobei 4,6 g, das sind 71 % der Theorie **Ethylcarbamat** mit einem Schmelzpunkt von 46 - 50°C erhalten wurden.

### Beispiel 14

Wie im Beispiel 13 beschrieben, aber unter Verwendung von 26,6 g 1-Hexadecanol (0,11 Mol) wurde nach Umkristallisieren aus Chloroform **Hexadecylcarbamat** in einer Ausbeute von 40 % der Theorie erhalten.
¹H-NMR: 0,89 (t, -CH₃); 1,2-1,6 (m, -CH₂-); 4,07 (t, -CH₂-O-); 7,2 (s, -NH₂).

### Beispiel 15

In 100 ml einer Lösung von 9,5 g (0,066 Mol) Triethylammomiumisocyanat in Chloroform, hergestellt nach Beispiel 1, wurde eine Lösung von 7 g (0,066 Mol) Benzylalkohol in 20 ml Chloroform bei Raumtemperatur unter Rühren getropft, worauf 12 Stunden bei Raumtemperatur nachgerührt und 30 Minuten auf Rückfluß erhitzt wurde.
Das Lösungsmittel wurde abgedampft und der Rückstand aus Ethanol umkristallisiert. Dabei wurden 7,0 g, das sind 70 % der Theorie **Benzylcarbamat** mit einem Schmelzpunkt von 88 - 89°C erhalten.

### Beispiel 16

Wie im Beispiel 13 beschrieben, aber unter Verwendung von 10,3 g 4-Chlorphenol (0,080 Mol) wurde nach dem Umkristallisieren aus Methanol/Wasser **4-Chlorphenylcarbamat**, in einer Ausbeute von 45 % der Theorie erhalten.
¹H-NMR: 5,5 (s, -NH₂); 6,8 (d, Aryl); 7,2 (d, Aryl)

### Beispiel 17

In 120 ml einer Lösung von 28 g (0,2 Mol) Triethylammoniumisocyanat in Chloroform, hergestellt nach Beispiel 1, wurden 18,5 g (0,3 Mol) Ethylmercaptan, gelöst in 30 ml Chloroform, bei 0°C unter Rühren getropft, worauf noch 1 Stunde bei dieser Temperatur und anschließend 15 Stunden bei Raumtemperatur nachgerührt wurde. Hierauf wurde die Reaktionsmischung 2 Stunden unter Rückfluß erhitzt. Das Lösungsmitel wurde abdestilliert. Der verbleibende ölige Rückstand kristallisierte beim Abkühlen und wurde aus Wasser umkristallisiert.
Dabei wurden 16 g (0.15 Mol), das sind 75 % der Theorie **Thiocarbamidsäure-S-ethylester** mit einem Schmelzpunkt von 99 - 102 °C erhalten

### Beispiel 18

Wie im Beispiel 17 beschrieben, aber unter Verwendung von 63 g 1-Octadecylmercaptan (0,22 Mol) gelöst in 30 ml N-Methylpyrrolidon wurde **Thiocarbamidsäure-S-octadecylester** in einer Ausbeute von 60 % der Theorie erhalten.

| | | | | |
|---|---|---|---|---|
| C-H-N-Analyse: | theoret.: | C 69,2 %, | H 11,9 %, | N 4,3 % |
| | gef.: | C 69,0 %, | H 12,0 %, | N 4,2 % |

### Beispiel 19

Wie im Beispiel 17 beschrieben, aber unter Verwendung von 18,3 g Isopropylmercaptan (0,24 Mol) wurde nach dem Umkristallisieren aus Chloroform **Thiocarbamidsäure-S-iso-propylester** in einer Ausbeute von 70 % der Theorie erhalten.

| | | | | |
|---|---|---|---|---|
| C-H-N-Analyse: | theoret.: | C 40,3 %, | H 7,5 %, | N 11,8 % |
| | gef.: | C 40,5 %, | H 7,3 %, | N 11,8 % |

### Beispiel 20

Wie im Beispiel 17 beschrieben, aber unter Verwendung von 11,3 g Ethandithiol (0,12 Mol) wurde nach dem Umkristallisieren aus Chloroform **1,2-Di(carbamoyl-thio)-ethan** in einer Ausbeute von 70 % der Theorie erhalten.
¹H-NMR: 2,91 (-CH₂-CH₂-); 7,56 (-NH₂)
IR: 1650 cm⁻¹, 1620 cm⁻¹

### Beispiel 21

In 100 ml einer Lösung von 64 g Triisopentylammoniumisocyanat (0,24 Mol) in Chloroform, hergestellt nach Beispiel 1, wurden unter Rühren bei 0 °C 100 ml einer Lösung von 10 g Cyclohexen (0,12 Mol) getropft, worauf 2 Stunden bei Raumtemperatur nachgerührt und anschließend ca. 4 Stunden auf Rückfluß erhitzt wurde.
Nach dem Abfiltrieren der entstandenen trüben Lösung wurde das Lösungsmittel abdestilliert und der Rückstand bei 68 bis 73 °C, 20 Torr destilliert.
Dabei wurden 7 g **Cyclohexylisocyanat**, das sind 46 % der Theorie, erhalten.

### Beispiel 22

In 100 ml einer Suspension von 11,3 g (0,078 Mol) Triethylammoniumisocyanat in Toluol, hergestellt nach der im Beispiel 1 beschriebenen Vorgangsweise, wurden unter Rühren 3 g (0,025 Mol) alpha-Methylstyrol getropft, worauf 3 Stunden bei Raumtemperatur nachgerührt und anschließend circa 4 Stunden auf Rückfluß erhitzt wurde.
Dabei wurde eine Lösung von alpha,alpha-Dimethylbenzylisocyanat in Toluol erhalten.
Nach Destillation bei 40 bis 45 °C, 1 Torr wurde alpha,alpha-**Dimethylbenzylisocyanat** in einer Ausbeute von 55 % der Theorie mit einem n_{D}²⁵ von 1,5048 erhalten.

### Beispiel 23

Zu einer Suspension von 16,22 g Triisopentylammoniumisocyanat (0,06 Mol) in 150 ml Toluol, hergestellt nach der im Beispiel 1 beschriebenen Vorgangsweise, wurden unter Rühren bei 0 °C 100 ml einer Lösung von 10 g m-Diisopropenylbenzol (0,06 Mol) in Toluol getropft, worauf 3 Stunden bei Raumtemperatur nachgerührt und 3 Stunden auf Rückfluß erhitzt wurde. Dabei wurde eine Lösung von m-Tetramethylxyloldiisocyanat in Toluol erhalten.
Nach der Destillation bei 90 bis 95 °C, 0,4 Torr wurde **m-Tetramethylxyloldiisocyanat** in einer Ausbeute von 50 % der Theorie mit einem n_{D}²⁵ von 1,5136 erhalten.

## Patentansprüche

1. Verfahren zur Herstellung unsymmetrisch substituierter Harnstoffe, Carbamate, Thiocarbamate oder substituierter Isocyanate, dadurch gekennzeichnet, daß ein Addukt aus Isocyansäure und einem tertiären Amin ein einem unter den Reaktionsbedingungen inerten Verdünnungsmittel mit einem primären oder sekundären Amin, einem Alkohol, einem Thiol oder einer Verbindung mit einer oder zwei nicht kumulierten olefinischen Doppelbindungen umgesetzt wird.

2. Verfahren nach Anspruch 1, dadurch gekennzeichnet, daß als Addukt aus Isocyansäure und einem tertiären Amin eine Verbindung der Formel R₁R₂R₃N.HNCO eingesetzt wird, in der R₁, R₂ und R₃ unabhängig voneinander eine geradkettige oder verzweigte Alkylgruppe, eine Aryl-, Alkylaryl oder Arylalkylgruppe bedeuten oder R₁R₂R₃N bedeutet einen cyclischen Aminteil.

3. Verfahren nach Anspruch 2, dadurch gekennzeichnet, daß ein Addukt aus Isocyansäure und einen Trialkylamin eingesetzt wird.

4. Verfahren nach einem der Ansprüche 1 bis 3, dadurch gekennzeichnet, daß als Verdünnungsmittel ein gegebenenfalls halogenierter, aliphatischer oder aromatischer Kohlenwasserstoff, oder ein Carbonsäureamid eingesetzt wird.

5. Verfahren nach Anspruch 4, dadurch gekennzeichnet, daß als Verdünnungsmittel Chloroform, Toluol oder N-Methylpyrrolidon eingesetzt wird.

6. Verfahren nach einem der Ansprüche 1 bis 5, dadurch gekennzeichnet, daß die Umsetzung bei Temperaturen von -20°C bis zum Siedepunkt des Verdünnungsmittels durchgeführt wird.

7. Verfahren nach Anspruch 6, dadurch gekennzeichnet, daß die Umsetzung bei -10 °C begonnen und beim Siedepunkt des Verdünnungsmittels vervollständigt wird.

8. Verfahren nach einem der Ansprüche 1 bis 7, dadurch gekennzeichnet, daß die Umsetzung unter Druck vervollständigt wird.

9. Verfahren nach einem der Ansprüche 1 bis 8, dadurch gekennzeichnet, daß das Addukt aus Isocyansäure und tertiärem Amin durch Umsetzung eines gasförmigen Gemisches einer Temperatur von 250 bis 600 °C aus Isocyansäure und Ammoniak mit einem tertiärem Amin und Abkühlen der gasförmigen Reaktionsmischung in einem inerten Verdünnungsmittel, wobei das Addukt aus Isocyansäure und tertiärem Amin kondensiert und vom gasförmigen Ammoniak abgetrennt wird, hergestellt ist.

10. Verfahren nach Anspruch 9, dadurch gekennzeichnet, daß das tertiäre Amin ein Trialkylamin ist.

## Claims

1. Process for the preparation of asymmetrically substituted ureas, carbamates, thiocarbamates or substituted isocyanates, characterized in that an adduct of isocyanic acid and a tertiary amine is reacted with a primary or secondary amine, an alcohol, a thiol or a compound having one or two non-cumulated olefinic double bonds in a diluent which is inert under the reaction conditions.

2. Process according to Claim 1, characterized in that, as the adduct of isocyanic acid and a tertiary amino, a compound of the formula R₁R₂R₃N.HNCO is employed in which R₁, R₂ and R₃ independently of one another denote a straight-chain or branched alkyl group, or an aryl, alkylaryl or arylalkyl group, or R₁R₂R₃N denotes a cyclic amino moiety.

3. Process according to Claim 2, characterized in that an adduct of isocyanic acid and a trialkylamine is employed.

4. Process according to one of Claims 1 to 3, characterized in that an optionally halogenated, aliphatic or aromatic hydrocarbon, or a carboxamide is employed as the diluent.

5. Process according to Claim 4, characterized in that chloroform, toluene or N-methylpyrrolidone is employed as the diluent.

6. Process according to one of Claims 1 to 5, characterized in that the reaction is carried out at temperatures from -20°C up to the boiling point of the diluent.

7. Process according to Claim 6, characterized in that the reaction is begun at -10°C and completed at the boiling point of the diluent.

8. Process according to one of Claims 1 to 7, characterized in that the reaction is completed under pressure.

9. Process according to one of Claims 1 to 8, characterized in that the adduct of isocyanic acid and tertiary amine is prepared by reaction of a gaseous mixture of isocyanic acid and ammonia at a temperature of 250 to 600°C with a tertiary amine and cooling of the gaseous reaction mixture in an inert diluent, the adduct of isocyanic acid and tertiary amine condensing and being separated from gaseous ammonia.

10. Process according to Claim 9, characterized in that the tertiary amine is a trialkylamine.

## Revendications

1. Procédé de préparation d'urées substituées asymétriquement, de carbamates, de thiocarbamates ou d'isocyanates substitués, caractérisé en ce qu'on fait réagir un produit d'addition de l'acide isocyanique et d'une amine tertiaire dans un diluant inerte dans les conditions de réaction, avec une amine primaire ou secondaire, un alcool, un thiol ou un composé avec une ou deux liaisons doubles oléfiniques non cumulées.

2. Procédé selon la revendication 1, caractérisé en ce qu'on utilise, comme produit d'addition d'acide isocyanique et d'une amine tertiaire, un composé de formule R₁R₂R₃N.HNCO, où R₁, R₂ et R₃ signifient chacun, indépendamment les uns des autres, un groupe alkyle à chaîne droite ou ramifiée, ou un groupe aryle, alkylaryle ou arylalkyle, ou R₁R₂R₃N signifie une partie d'amine cyclique.

3. Procécé selon la revendication 2, caractérisé en ce qu'on utilise un produit d'addition d'acide isocyanique et d'une trialkylamine.

4. Procédé selon l'une des revendications 1 à 3, caractérisé en ce qu'on utilise, comme diluant, un hydrocarbure aliphatique ou aromatique, éventuellement halogéné, ou un carboxamide.

5. Procédé selon la revendication 4, caractérisé en ce qu'on utilise, comme diluant, le chloroforme, le toluène ou la N-méthylpyrrolidone.

6. Procédé selon l'une des revendications 1 à 5, caractérisé en ce qu'on effectue la réaction à des températures comprises entre -20°C et le point d'ébullition du diluant.

7. Procédé selon la revendication 6, caractérisé en ce que la réaction commence à -10°C et est complétée au point d'ébullition du diluant.

8. Procédé selon l'une des revendications 1 à 7, caractérisé en ce qu'on effectue la réaction sous pression.

9. Procédé selon l'une des revendications 1 à 8, caractérisé en ce qu'on prépare le produit d'addition d'acide isocyanique et d'amine tertiaire en faisant réagir un mélange gazeux d'acide isocyanique et d'ammoniac, à une température de 250 à 600°C, avec une amine tertiaire, et en refroidissant le mélange réactionnel gazeux dans un diluant inerte, le produit d'addition d'acide isocyanique et d'amine tertiaire étant condensé et séparé de l'ammoniac.

10. Procédé selon la revendication 9, caractérisé en ce que l'amine tertiaire est une trialkylamine.
